(19) Europäisches Patentamt
European Patent Office
Office européen des brevets

(11) Veröffentlichungsnummer: **0 217 737 B1**

(12) **EUROPÄISCHE PATENTSCHRIFT**

(45) Veröffentlichungstag der Patentschrift: **14.08.91**

(51) Int. Cl.[5]: **C07D 471/04**, A61K 31/435, //(C07D471/04,221:00,209:00)

(21) Anmeldenummer: **86730154.1**

(22) Anmeldetag: **03.10.86**

Die Akte enthält technische Angaben, die nach dem Eingang der Anmeldung eingereicht wurden und die nicht in dieser Patentschrift enthalten sind.

(54) **Tetrahydro-beta-carboline, Verfahren zu ihrer Herstellung und ihre Verwendung als Arzneimittel.**

(30) Priorität: **04.10.85 DE 3535928**

(43) Veröffentlichungstag der Anmeldung:
**08.04.87 Patentblatt 87/15**

(45) Bekanntmachung des Hinweises auf die Patenterteilung:
**14.08.91 Patentblatt 91/33**

(84) Benannte Vertragsstaaten:
**AT BE CH DE ES FR GB GR IT LI LU NL SE**

(56) Entgegenhaltungen:
**EP-A- 0 030 254**
**FR-M- 1 825**

**JOURNAL CHEMICAL SOCIETY, Sektion C, 1970, Seiten 303-307; A.H. KELLY et al.: "Diazaindenes (Azaindoles). part II.1 thermal indolisation of 3- and 4-pyridylhydrazones"**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Biere, Helmut, Dr.**
**Zeltinger Strasse 15**
**W-1000 Berlin 28(DE)**
Erfinder: **Engelstoft, Mogens**
**Mosegard Park 121**
**DK-3500 Vaerlose(DK)**
Erfinder: **Huth, Andreas, Dr.**
**Kirchweg 55**
**W-1000 Berlin 38(DE)**
Erfinder: **Rahtz, Dieter, Dr.**
**Krottnaurerstrasse 24 a**
**W-1000 Berlin 38(DE)**
Erfinder: **Schmiechen, Ralph, Dr.**
**Bayernring 27**
**W-1000 Berlin 42(DE)**
Erfinder: **Seidelmann, Dieter, Dr.**
**Stierstrasse 14**
**W-1000 Berlin 41(DE)**
Erfinder: **Stephens, David Norman, Dr.**
**Hildegardstrasse 16a**
**W-1000 Berlin 31(DE)**

EP 0 217 737 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 5,6,7,8-Tetrahydro-β-carboline, ihre Herstellung und ihre Verwendung als Arzneimittel.

Die erfindungsgemäßen Verbindungen haben die allgemeine Formel I

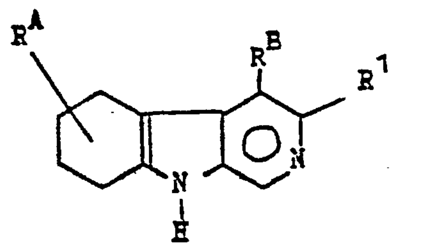

(I)

worin

$R^1$ einer Oxadiazolylrest der Formel

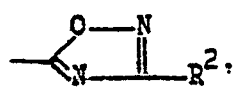

mit $R^2$ in der Bedeutung von E, niederem Alkyl oder Cycloalkyl, und eine $COOR^3$-Gruppe mit $R^3$ in der Bedeutung von H oder niederem Alkyl und eine $CONR^4R^5$-Gruppe mit $R^4$ und $R^5$ in der Bedeutung von H oder niederem Alkyl, Hydroxy-nieder Alkyl, wobei $R^4$ und $R^5$ nicht gleichzeitig Wasserstoff sein können, oder $R^4$ und $R^5$ gemeinsam mit dem benachbarten Stickstoffatom einen 5- oder 6-gliedrigen Ring bilden, der zusätzlich ein Heteroatom enthalten kann, darstellt, und

$R^A$ Wasserstoff, =O, Cycloalkyl, $-COOR^3$ mit $R^3$ in der oben genannten Bedeutung, oder niederes Alkyl, das gegebenenfalls durch OH, Halogen, niederes Alkoxy, Phenyl, Phenyloxy, $-NR^4R^5$, wobei $R^4$ und $R^5$ die oben genannte Bedeutung haben, substituiert sein kann, bedeutet und

$R^B$ Wasserstoff, niederes Alkyl oder niederes Alkoxyalkyl sein kann.

Es ist bekannt, daß die Wirkungsstärke der β-Carboline von der Intensität ihrer Bindung an den Benzodiazepinrezeptor abhängt (C. Braestrup, M. Nielsen J. Neurochem. 37, 333-341 (1981)). Ferner ist bekannt, daß für eine hohe Affinität an den Benzodiazepinrezeptor ein planares aromatisches System erforderlich ist. So erwies sich beispielsweise Norharman-3-carbonsäureethylester als erheblich wirksamer als 1,2,3,4-Tetrahydro-β-carbolin-3-carbonsäureethylester (H.A. Robertson et al. Eur. J. Pharmacol. 76, 281-284 (1981)).

Daher war nicht zu erwarten, daß bei Aufgabe des planaren Systems der β-Carboline Verbindungen erhalten werden, die eine hohe Affinität zum Benzodiazepinrezeptor besitzen.

Es wurde nun gefunden, daß die neuen, 5,6,7,8-Tetrahydro-β-carboline der allgemeinen Formel I eine den entsprechenden im A-Ring aromatischen β-Carbolinderivaten vergleichbare Affinität zum Benzodiazepinrezeptor besitzen.

Der Substituent $R^A$ der neuen β-Carboline kann in 5-, 6-, 7- oder 8-Stellung stehen, wobei die Substitution in 5- oder 6-Stellung bevorzugt ist und der Substituent $R^A$ ein- oder mehrfach vorhanden ist.

Unter niederem Alkyl sind Alkylreste mit 1 - 4 Kohlenstoffatomen zu verstehen wie beispielsweise Methyl, Ethyl, n-Propyl, i-Propyl, n-Butyl, tert.-Butyl.

Der Cycloalkylrest kann 3-6 C-Atome haben wie beispielsweise Cyclopropyl, Cyclobutyl, Cyclopentyl, Cyclohexyl, wobei für $R^2$ als Cycloalkylrest Cyclopropyl bevorzugt ist.

Bilden $R^4$ und $R^5$ gemeinsam mit dem Stickstoffatom einen Heterocyclus, so ist dieser 5 - 6 gliedrig und die Alkylenbrücke kann zusätzlich ein N-, O- oder S-Atom enthalten, wobei Sauerstoff bevorzugt ist.

Die folgenden Heterocyclen seien als Beispiele genannt: Piperidin, Morpholin, Pyrrolidin, Thiomorpholin, Piperazin usw.

Als niedere Alkylreste $R^A$ sind bevorzugt solche mit bis zu 2 Kohlenstoffatomen anzusehen.

Unter Halogen ist vorzugsweise Chlor, Brom oder Jod zu verstehen.

Die erfindungsgemäßen Verbindungen gemäß der Formel I zeigen überraschenderweise im Vergleich

zu 1,2,3,4-Tetrahydro-$\beta$-carbolinen überlegene und im Vergleich zu den aromatischen Verbindungen im pharmakologischen Test mindestens gleich gute psychotrope Eigenschaften.

Die pharmakologischen Eigenschaften der erfindungsgemäßen Verbindungen wurden durch Untersuchung des Verdrängungsvermögens von radioaktiv-markiertem Flunitrazepam von Benzodiazepin-Rezeptoren bestimmt.

Die Verdrängungsaktivität der erfindungsgemäßen Verbindungen wird als $IC_{50}$- und $ED_{50}$- Wert angegeben. Der $IC_{50}$-Wert gibt die Konzentration an, die eine 50 %ige Verdrängung der spezifischen Bindung von $^3$H-Flunitrazepam (1,0 nM, 0 °C) in Proben mit einen Gesamtvolumen von 0,55 ml einer Suspension von Hirnmembranen, z.B. von Ratten.

Der Verdrängungstest wird wie folgt ausgeführt:
0,5 ml einer Suspension von unbehandeltem Rattenhirn in 25 mM $KH_2PO_4$, pH = 7,1 (5 - 10 mg Gewebe/Probe) wird für 40 - 60 Minuten bei 0 °C zusammen mit $^3$H-Diazepam (spezifische Aktivität 14,4 Ci/mmol, 1,9 nM) oder $^3$H-Flunitrazepam (spezifische Aktivität 87 Ci/mmol, 1,0 nM) inkubiert. Nach Inkubation wird die Suspension durch eine Glasfritte filtriert, der Rückstand zweimal mit kalter Pufferlösung gewaschen und die Radioaktivität im Scintillationszähler gemessen.

Der Versuch wird dann wiederholt, jedoch so, daß vor der Zugabe des radioaktiv-markiertem Benzodiazepins eine bestimmte Menge oder eine überschüssige Menge der Verbindung, deren Verdrängungsaktivität zu bestimmen ist, zugesetzt wird. Auf Grundlage der erstimmen ist, zugesetzt wird. Auf Grundlage der erhaltenen Werte kann dann der $IC_{50}$-Wert berechnet

Der $ED_{50}$-Wert stellt die Dosis einer Versuchssubstanz dar, die eine Reduktion der spezifischen Bindung des Flunitrazepams an dem Benzodiazepin-Rezeptor in einem lebenden Gehirn auf 50 % des Kontrollwertes bewirkt.

Der in-vivo Test wird wie folgt ausgeführt:
Gruppen von Mäusen wird die Versuchssubstanz bei unterschiedlichen Dosen und normalerweise intraperitoneal injiziert. Nach 15 Minuten wird en Mäusen das $^3$H-Flunitrazepam intravenös verabreicht. Nach weiteren 20 Minuten werden die Mäuse getötet, ihr Vorderhirn entfernt und die Radioaktivität der Vorderhirne durch Scintillationszählung gemessen. Der $ED_{50}$-Wert wird aus den Dosis/Wirkungs-Kurven bestimmt.

Aufgrund der Benzodiazepin-Rezeptor-Affinität zeigen die erfindungsgemäßen Verbindungen die von den Benzodiaxepinen bekannten Eigenschaften wie beispielsweise Muskel-Relaxation, anti-aggressiv anxiolytisch oder antikonvulsiv. Die erfingungsgemäßen Verbindungen sind auch geeignet zur Behandlung von Schlafstörungen und von Gedächtnisstörungen.

Die neuen Verbindungen der allgemeinen Formel I besitzen wertvolle pharmakologische Eigenschaften. Insbesondere wirken sie auf das Zentralnervensystem und sind somit als Psychopharmaka in der Humanmedizin geeignet.

Die erfindungsgemäßen Verbindungen können zur Formulierung von pharmazeutischen Präparationen, z.B. für die orale und parenterale Anwendung bei Säugetieren einschließlich Menschen, nach an sich bekannten Methoden der Galenik verwendet werden.

Als Hilfsstoffe zur Formulierung von pharmazeutischen Als Hilfsstoffe zur Formulierung von pharmazeutischen Präparationen sind solche physiologisch verträglichen organischen und anorganischen Trägersubstanzen für die enterale und parenterale Anwendung geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermittel, Konservierungsstoffen, Stabilisatoren, Netzmittel, Emulgatoren, Puffermittel und Farbstoffen, versetzt werden.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Für die orale Anwendung sind insbesondere Tabletten, Dragees oder Kapseln mit Talkum und/oder einem Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 100mg aktiver Substanz in einem physiologisch verträglichen Träger eingebracht.

Die erfindungsgemäßen Verbindungen werden in einer Dosis von 1,1 bis 300 mg/Tag, vorzugsweise 1-30 mg/Tag, angewendet.

Die Herstellung der erfindungsgemäßen Verbindungen erfolgt nach an sich bekannten Verfahren, indem man eine Verbindung der allgemeinen Formel II

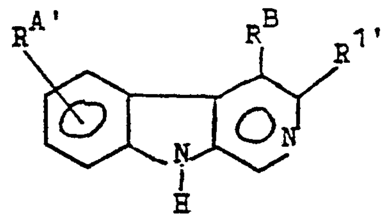

(II),

worin

- $R^B$ die oben genannte Bedeutung hat,
- $R^{1'}$ -COOR³ darstellt mit $R^3$ in der oben genannten Bedeutung und
- $R^{A'}$ Wasserstoff, eine Hydroxygruppe, Cycloalkyl, -$COOR^3$, worin $R^3$ die oben genannte Bedeutung hat, oder niederes Alkyl, das mit Phenyl, niederem Alkoxy oder Hydroxy substituiert sein kann, bedeutet, in Gegenwart eines Katalysators hydriert und gegebenenfalls anschließend Verbindungen der allgemeinen Formel I mit $R^A$ in der Bedeutung einer Hydroxyalkylgruppe mit Phenol veräthert oder die Hydroxygruppe durch sekundäre Amine oder durch Halogen substituiert und anschließend gegebenenfalls die so erhaltene Halogenalkylverbindung mit einem Amin der Formel $HNR^4R^5$ mit $R^4$ und $R^5$ in der oben genannten Bedeutung aminiert oder eine Estergruppe umestert, amidiert oder hydrolisiert und gegebenenfalls anschließend die so erhaltene freie Säure amidiert oder in den tert.-Butylester überführt oder mit einem Amidoxim der Formel $R^2$-C(-NOH)$NH_2$ mit $R^2$ in der oben genannten Bedeutung umsetzt.

Die Hydrierung wird in inerten Lösungsmitteln, zum Beispiel in Alkoholen, wie Methanol, Ethanol, Propanol, Butanol oder in Säuren wie Essigsäure oder in Ethern wie Dioxan, Diethylether in Gegenwart eines Katalysators vorgenommen.

Um Umesterungen zu vermeiden, nimmt man zweckmäßigerweise den entsprechenden Alkohol als Lösungsmittel.

Als Katalysatoren sind alle üblichen Hydrierungskatalysatoren geeignet wie beispielsweise Raney-Nickel oder Edelmetallkatalysatoren wie Palladium oder Platin, gegebenenfalls auf geeigneten Trägern wie Kohle oder Calciumcarbonat.

Im allgemeinen gibt man bei Edelmetallkatalysatoren etwas Säure wie Essigsäure zu, um die Reaktion zu Beschleunigen.

Die Hydrierung kann bei Normaldruck oder $H_2$-Druck bis zu 100 bar durchgeführt werden.

Die Reaktionstemperatur kann von Raumtemperatur bis auf 100 °C (bei Arbeiten unter Druck) erhöht werden. Nach ca. 2-6 Stunden ist die Reaktion im allgemeinen beendet.

Die nach der Hydrierung gegebenenfalls vorgenommen Umsetzungen erfolgen alle nach an sich bekannten Verfahren.

Beispielsweise wird die Hydroxyalkylgruppe $R^A$ halogeniert, indem man zur Einführung von Brom mit Phosphortribromid oder HBr umsetzt, zur Einführung von Chlor mit Thionylchlorid umsetzt und zur Einführung von Jod mit NaJ das entsprechende Bromid oder Chlorid umsetzt.

Die Halogenierungsreaktion kann unter Kühlung auf - 10 °Cbis zum Siedepunkt das Lösungsmittels durchgeführt werden.

Als Lösungsmittel sind alle inerten, bevorzugt aprotischen, Lösungsmittel geeignet wie beispielsweise chlorierte Kohlenwasserstoffe wie Dichloräthan, Dichlormethan, Chloroform oder Ether wie Diethylether, Tetrahydrofuran, Dioxan und Ketone wie Aceton u.a.

Auch nach der von J.R. Falck und Sukumar Manna in Synthetic Communications, 15 (8) 663 - 8 (1985) beschriebenen Methode kann die Halogenierung vorgenommen werden.

Die so erhaltene Halogenalkyl-β-carboline können beispielsweise mit primären oder sekundären Aminen der Formel $HNR^4R^5$, mit $R^4$ und $R^5$ in der oben genannten Bedeutung, in die entsprechenden Aminoalkyl-β-carbolinderivate überführt werden.

Gewünschtenfalls kann das bei der Hydrierung entstehende. Stereoisomerengemisch nach den üblichen Methoden beispielsweise chromatographisch in die Antipoden getrennt werden.

Die Herstellung von Phenoxyalkylderivaten kann beispielsweise mit Triphenylphosphin, Azodicarbonsäurediethylester und Phenol erfolgen. (M.S. Manhas et al. J. of the Chemical Society London 1975. Seite 461 - 463). Analog können nach diesem Verfahren unter Verwendung von sekundären Aminen die entsprechenden Aminoalkylderivate hergestellt werden.

Die sich gegebenenfalls anschließende Verseifung einer Estergruppe in 3-Stellung oder im A-Ring erfolgt zweckmäßigerweise alkalisch, wobei der Ester mit verdünnter wässriger Alkalilauge wie Kalium- oder Natriumhydroxid, in einem protischen Lösungsmittel, wie zum Beispiel Methanol, Ethanol oder Ethylenglykol, auf Temperaturen bis zur Rückflußtemperatur des Reaktionsgemisches erhitzt wird.

Die so erhaltenen freien β-Carbolin-carbonsäuren dienen z.B. zur Einführung des 5-Oxadiazolylrestes. Hierzu wird die β-Carbolincarbonsäure mit einem Amidoxim der Formel $R^2$-C(=NOH)$NH_2$, in einem inerten Lösungsmittel, das über 100 °C siedet und gegenüber den Reaktanten inert ist, bei der Rückflußtemperatur des Reaktionsgemisches zur Kondensation gebracht. Geeignete Lösungsmittel für die Kondensationsreaktion sind beispielsweise Toluol und Dimethylformamid. Zweckmäßigerweise wird die freie β-Carbolin-3-carbonsäure vor der Kondensationsreaktion in geeigneter Weise aktiviert. Hierzu kann die freie Säure in das gemischte Anhydrid, in den aktivierten Ester oder in das Chlorid überführt werden.

Gut bewährt hat sich eine Aktivierung mit Imidazol/Thionylchlorid in einem aprotischen Lösungsmittel wie Dioxan, Tetrahydrofuran, Dimethylformamid oder N-Methylpyrrolidon bei Temperaturen zwischen 0 und 50 °C, vorzugsweise Raumtemperatur.

Wird eine Umesterung gewünscht, so kann man mit dem entsprechenden Alkohol oder Alkalialkoholat umsetzen, gegebenenfalls kann man Titantetra-isopropylat als Katalysator zufügen. Üblicherweise wird die Umesterung bei Temperaturen von 60 bis 120 °C durchgeführt und ist nach ca. 2 - 6 Stunden beendet.

Die Einführung der tert.-Butylestergruppe erfolgt beispielsweise durch Umsetzung der Carboxylgruppe mit tert.-Butoxy- bis-dimethyl-aminomethan. Im allgemeinen wird die Reaktion unter Inertgasatmosphäre wie Argon oder Stickstoff durchgeführt.

Aus den oben beschriebenen β-Carbolincarbonsäuren lassen sich in bekannter Weise beispielsweise über die Säurehalogenide durch Umsetzung mit primären oder sekundären Aminen die entsprechenden Amide herstellen.

Die durch Umsetzung der Säuren mit 1,1'-Carbonyldimidazol erhältlichen Imidazolide sind ebenfalls geeignete Vorstufen für die Darstellung der Amide (Klaus P. Lippke et al. J. Pharmaceutical Sci. 74, 676 - 680 (1985) ).

Die nachfolgenden Beispiele sollen das erfindungsgemäße Verfahren erläutern.

Die Herstellung der Ausgangsverbindungen ist bekannt oder erfolgt nach an sich bekannten Verfahren.

Beispiel 1

5.6.7.8-Tetrahydro-5-ethoxymethyl-4-methyl-β-carbolin-3-carbonsäureethylester

5-Ethoxymethyl-4-methyl-β-carbolin-3-carbonsäureethylester(1 g) wird in Ethanol (80 ml) mit 10%iger Palladiumkohle (0,3 g) bei einem Wasserstoffdruck von 40 bar und einer Temperatur von 70 °C 2 Stunden geschüttelt. Nach Abfiltrieren des Katalysators wird die Reaktionslösung eingedampft, der Rückstand Kristallisiert beim Behandeln mit einem Gemisch aus Essigester und Ethanol. Die Ausbeute an 5,6,7,8-Tetrahydro-5-ethoxymethyl-4-methyl-β-carbolin-3-carbonsäureethylester beträgt 0,66 g.
Schmelzpunkt 222 - 224 °C

Analog werden dargestellt:

5,6,7,8-Tetrahydro-4,5-dimethyl-β-carbolin-3-carbonsäureethylester. Schmelzpunkt 103-105 °C.

5,6,7,8-Tetrahydro-5-methoxymethyl-4-methyl-β-carbolin-3-carbonsäureethylester. Schmelzpunkt 200 - 202 °C.

5,6,7,8-Tetrahydro-β-carbolin-3,5-dicarbonsäurediethylester. Schmelzpunkt 214 - 216 °C.

5,6 7,8-Tetrahydro-4-methyl-5-propoxymethyl-β-carbolin-3-carbonsäureethylester

5,6,7,8-Tetrahydro-4-methoxymethyl-β-carbolin-3-carbonsäureethylester.(Schmelzpunkt 174 - 175 °C aus $CH_2Cl_2$/Aceton)

5, 6, 7, 8-Tetrahydro-4-methyl-5-oxo-β-carbolin-3-carbonsäure-ethyl-ester. Schmelzpunkt 204 - 206 °C.

5, 6, 7, 8-Tetrahydro-5-ethoxymethyl-4-methoxym ethyl-β-carbolin-3-carbonsäure-ethylester.

5, 6, 7, 8-Tetrahydro-5-ethoxymethyl-β-carbolin-3-carbonsäure-ethyl-ester. Schmelzpunkt 190 - 191 °C (aus Ethanol/Ether).

Ausgehend von den vollaromatischen Isopropylestern wurden in Isopropanol anstelle von Ethanol, aber sonst gemäß Beispiel 1 hergestellt:

5,6,7,8-Tetrahydro-5-ethyl-β-carbolin-3-carbonsäure-isopropylester. Schmelzpunkt 191 - 193 °C (aus Diisopropylether).

5, 6, 7, 8-Tetrahydro-5-(1-hydroxyethyl)-β-carbolin-3-carbonsäure-iso-propylester. Schmelzpunkt 177 - 179 °C (aus Diisopropylether).

5,6,7, 8-Tetrahydro-5-ethoxymethyl-4-ethyl-β-carbolin-3-carbonsäure-ethylester Schmelzpunkt 273 - 275 °C.

5,6,7,8-Tetrahydro-β-carbolin-3-carbonsäureethylester. Schmelzpunkt 224 - 226 °C.

5,6,7,8-Tetrahydro-4-ethyl-β-carbolin-3-carbonsäureethylester (Schmelzpunkt 198 - 200 °C aus Hexan/Aceton)

5,6,7,8-Tetrahydro-4,5-dimethoxymethyl-β-carbolin-3-carbonsäureethylester

Beispiel 2

5.6.7.8-Tetrahydro-5-hydroxymethyl-4-methoxymethyl-β-carbolin-3-carbonsäureethylester

5-Hydroxymethyl-4-methoxymethyl-β-carbolin-3-carbonsäure-ethylester (0,5 g) werden in 80 ml Ethanol mit Raney-Nickel unter Wasserstoff bei Normaldruck und Raumtemperatur 6 Std. geschüttelt. Aufarbeitung wie in Beispiel 1. Die Ausbeute an 5,6,7,8-Tetrahydro-5-hydroxymethyl -4-methoxymethyl-β-carbolin-3-carbonsäureethylester beträgt 0,3 g.

Analog wird dargestellt:

5,6,7,8-Tetrahydro-5-hydroxymethyl-4-methyl-β-carbolin-3-carbonsäureethylester

Beispiel 3

510 mg 4-Methyl-β-carbolin-3-carbonsäureethylester werden in 27 ml Ethanol und 3 ml Eisessig mit 400 mg Palladium Mohr bei einem Wasserstoffdruck von 10 bar bei 75 °C 5 Stunden hydriert. Nach Abfiltrieren des Katalysators wird eingedampft und zwischen Methylenchlorid und gesättigter Natriumbicarbonatlösung verteilt. Die organische Phase wird mit gesättigter Kochsalzlösung gewaschen, getrocknet, filtriert und eingeengt. Nach Chromatographie über Kieselgel mit Methylenchlorid/Ethanol = 5/1 und Umkristallisation aus Ethanol/Hexan erhält man 130 mg 5,6,7,8-Tetrahydro-4-methyl-β-carbolin-3-carbonsäureethylester vom Schmelzpunkt 244 - 245 °C.

In analoger Weise werden aus 6-Benzyl-4-methyl-β-carbolin-3-carbonsäureethylester:

5,6,7,8-Tetrahydro-6-benzyl-4-methyl-β-carbolin-3-carbonsäureethylester (Schmelzpunkt 196 - 197 °C aus Ethanol/Hexan)

und aus 6-Cyclohexyl-4-methyl-β-carbolin-3-carbonsäureethylester:

5,6,7,8-Tetrahydro-6-cyclohexyl-4-methyl-β-carbolin-3-carbonsäureethylester (Schmelzpunkt 198 - 201 °C) hergestellt.

Beispiel 4

520 mg 5,6,7,8-Tetrahydro-4-methoxymethyl-β-carbolin-3-carbonsäure werden in 20 ml Dimethylformamid mit 32 ml einer frisch bereiteten 0,25 molaren Lösung von Thionyldiimidazol in Tetrahydrofuran versetzt und 1 Stunde bei Raumtemperatur gerührt. Die entstandene klare Lösung wird mit 2,11 g Propionamidoxim versetzt und 2,5 Stunden bei Raumtemperatur gerührt. Nach Stehen über Nacht wird eingeengt, in 30 ml Toluol aufgenommen und 2 Stunden am Rückfluß gekocht. Nach Zugabe von 50 ml Wasser wird geschüttelt bis eine abgeschiedene braune Schmiere gelöst ist und die Toluolphase abgetrennt. Die wässrige Phase wird dreimal mit je 50 ml Essigester extrahiert und die gesammelte organische Phase eingeengt. Dr Rückstand wird zunächst aus Essigester/Cyclohexan und dann aus Ethanol/Essigester umkristallisiert.

Man erhält 147 mg 3-(3-Ethyl-1,2,4-oxadiazol-5-yl-)-4-methoxymethyl-5,6,7,8-tetrahydro-ß-carbolin vom Schmelzpunkt 197 - 199 °C.

Beispiel 5

0,7 g 5-Hydroxy-4-methoxymethyl-ß-carbolin-3-carbonsäureethylester werden in 50 ml Ethanol mit 0,5 g Raney-Nickel bei einem Wasserstoffdruck von 80 bar und einer Temperatur von 100 °C 6 Stunden hydriert. Nach Abfiltrieren des Katalysators wird das Lösungsmittel im Vakuum eingedampft. Der Rückstand wird an Kieselgel mit Dichlormethan und Ethanol = 10 + 1 chromatographiert. Man erhält 0,108 g 4-Methoxymethyl-5-oxo-5,6,7,8,-tetrahydro-β-carbolin-3-carbonsäureethylester von Schmelzpunkt 249 - 250 °C.

Beispiel 6

5.6.7.8-Tetrahydro-5-brommethyl-4-methoxymethyl-β-carbolin-3-carbonsäureethylester

Aus 5,6,7,8-Tetrahydro-5-hydroxymethyl-4-methoxymethyl-β-carbolin-3-carbonsäureethylester (Beispiel 2) durch Umsetzung mit Phosphortribromid in Dichlormethan.

Beispiel 7

5,6,7,8-Tetrahydro-5-brommethyl-4-methoxymethyl-β-carbolin-3-carbonsäureethylester (0,25 g) in Dichlormethan (10 ml) wird mit einer Lösung von Morpholin (1,0 ml) in Ethanol (5 ml) versetzt. Das Gemisch wird am Rückfluß gekocht. Nach dem Abdampfen der Lösungsmittel läßt sich aus dem Rückstand durch Chromatographie an Kieselgel mit einem Gemisch aus Dichlormethan ( 19 Teile) und Ethanol (1 Teil) 5,6,7,8-Tetrahydro-4-methoxymethyl-5-(4-morpholinyl)methyl-β-carbolin-3-carbonsäure-ethylester (0,1 g) isolieren. Schmelzpunkt 188 - 191 °C.

Analog werden hergestellt:
5,6,7,8-Tetrahydro-5-diethylaminomethyl-4-methoxymethyl-β-carbolin-3-carbonsäureethylester
5,6,7,8-Tetrahydro-5-diethanolaminomethyl-4-methoxymethyl-β-carbolin-3-carbonsäureethylester
5,6,7,8-Tetrahydro-5-isopropylaminomethyl-4-methoxymethyl-β-carbolin-3-carbonsäureethylester.

Beispiel 8

0,5 g 5,6,7,8-Tetrahydro-5-ethoxymethyl-4-methyl-β-carbolin-3-carbonsaüreethylester werden in Ethanol (40 ml) mit 1-normaler Natronlauge (3,5 ml) 4 Stunden am Rückfluß gekocht. Nach dem Erkalten wird 1-normale Essigsäure (3,5 ml) zugesetzt und eingedampft. Der Eindampfrückstand wird in Wasser suspendiert, abfiltriert und gut mit Wasser gewaschen. Man erhält 0,3 g 5,6,7,8-Tetrahydro-5-ethoxymethyl-4-methyl-β-carbolin-3-carbonsäure.

Analog werden hergestellt:
5,6,7,8-Tetrahydro-4-methoxymethyl-5-(4-morpholinyl) methyl-β-carbolin-3-carbonsäure
5,6,7,8-Tetrahydro-4-methyl-5-(4-morpholinyl) methyl-β-carbolin-3-carbonsäure

Beispiel 9

5.6.7.8-Tetrahydro-5-ethoxymethyl-4-methyl-β-carbolin-3-carbonsäure-tert.-butylester

0,35 g 5,6,7,8-Tetrahydro-5-ethoxymethyl-4-methyl-β-carbolin-3-carbonsäure werden in tert.-Butoxy-bis-(dimethylamino)methan (8 ml) in einer Argonatomosphäre 3 Stunden auf 120 °C erwärmt. Nach Abdampfen der flüchtigen Bestandteile wird der Rückstand an Kieselgel mit einer Mischung aus Dichlormethan (8 Teile), Aceton ( 1 Teil) und Ethanol (1 Teil) chromatographiert. Die Ausbeute beträgt 0,2 g.

Beispiel 10

0,2 g 4-Methoxymethyl-5-oxo-5,6,7,8-tetrahydro-β-carbolin-3-carbonsäureethylester werden in 10 ml Isopropanol mit 0,1 ml Titan-tetraisopropylat 1,5 Stunden unter Argonatmosphäre am Rückfluß gekocht. Die nach dem Abkühlen ausgefallenen Kristalle werden abgesaugt und mit Isopropanol nachgewaschen. Man erhält 0,135 g 4-Methoxymethyl-5-oxo-5,6,7,8-tetrahydro-β-carbolin-3-carbonsäureisopropylester vom Schmelzpunkt 243 - 245 °C.

Beispiel 11

1,15 g 5,6,7,8-Tetrahydro-4-methoxymethyl-β-carbolin-3-carbonsäureethylester werden in 45 ml Ethanol mit 5 ml 2n-wässriger Kaliumhydroxidlösung 2 Stunden am Rückfluß gekocht. Nach Abkühlen wird mit Eisessig sauer gestellt. Das ausgefallene Produkt wird abgesaugt; die Mutterlauge wird auf ~ 30 ml eingeengt. Das ausgefallene Produkt wird wiederum abgesaugt. Man erhält 1 g 5,6,7,8-Tetrahydro-4-methoxymethyl-β-carbolin-3-carbonsäure vom Schmelzpunkt > 260 °C.

Analog werden hergestellt:
5,6,7,8-Tetrahydro-4,5-dimethyl-β-carbolin-3-carbonsäure
5,6,7,8-Tetrahydro-5-methoxymethyl-4-methyl-β-carbolin-3-carbonsäure

Beispiel 12

5.6.7.8-Tetrahydro-5-ethoxymethyl-3-(3-ethyl-1.2.4-oxadiazol-5-yl)-4-methyl-β-carbolin

Zu einer Suspension von 5,6,7,8-Tetrahydro-5-ethoxymethyl-4-methyl-β-carbolin-3-carbonsäure (0,36 g) in Tetrahydrofuran (10 ml) wird Thionyldimidazolid (~2,5 ml) in Tetrahydrofuran (10 ml) gefügt. Nach einer Stunde wird Propionamidoxim (0,4 g) zur Lösung gegeben. Nach 2 Stunden bei Raumtemperatur wird die Mischung im Vakuum eingeengt, Wasser (25 ml) zugefügt und die Lösung mit Dichlormethan extrahiert. Die organische Phase wird in Vakuum eingedampft, der Rückstand wird mit p-Xylol (25 ml) unter Stickstoff 2 Stunden auf 160 °C erhitzt. Der nach Einengen im Vakuum verbleibende Rückstand wird an Kieselgel mit Methylenchlorid chromatographiert.

5,6,7,8-Tetrahydro-5-ethoxymethyl-3-(3-ethyl-1,2,4,-oxadiazol-5-yl)-4-methyl-β-carbolin (0, 10 g) wird in Form farbloser Kristalle durch Behandlung mit Petrolether erhalten.

Analog werden hergestellt:

5,6,7,8-Tetrahydro-4,5-dimethyl-3-(3-ethyl-1,2,4-oxadiazol-5-yl-)-β-carbolin

5,6,7,8-Tetrahydro-3-(3-ethyl-1,2,4-oxadiazol-5-yl)-5-methoxymethyl-4-methyl-β-carbolin

Analog werden hergestellt:

5,6,7,8-Tetrahydro-5-ethoxymethyl-3-(3-ethyl-1,2,4-oxadiazol-5-yl)-4- methoxymethyl-β-carbolin. Schmelzpunkt 171 - 173 °C.

5,6,7,8-Tetrahydro-3-(3-ethyl-1,2,4-oxadiazol-5-yl)-4-methoxymethyl-5-(4-morpholinyl)methyl -β-carbolin.

Beispiel 13

5,6,7,8-Tetrahydro-3-(3-ethyl-1,2,4-oxadiazol-5-yl)-4,5-dimethoxy-methyl-β-carbolin

zu einer Suspension von 5,6,7,8-Tetrahydro-4,5-dimethoxymethyl-β-carbolin-3-carbonsäure (0,55 g) in absolutem Dimethylformamid (40 ml) wird bei Raumtemperatur Carbonyldiimidazol (0,85 g) gefügt. Das Gemisch wird erst 24 Stunden bei Raumtemperatur und anschließend 5 Stunden bei 60 °C gerührt. Anschließend wird bei Raumtemperatur Propionamidoxin (0,64 g) zugefügt. Nach 4 Stunden wird das Gemisch eingedampft. Der Eindampfungsrückstand wird in Xylol am Wasserabscheider 3 Stunden am Rückfluß gekocht. Nach Stehen über Nacht wird vom ungelösten dekantiert, das ungelöste wird noch zweimal mit Xylol extrahiert. Die vereinigten Extrakte und die ursprüngliche Lösung werden eingedampft, der Rückstand wird an Kieselgel mit einem Gemisch aus 95 Teilen Dichlormethan und 5 Teilen Methanol chromatographiert und ergibt nach umkristallisieren aus Ethanol das gewünschte 5,6,7,8-Tetrahydro-3-(3-ethyl-1,2,4-oxadiazol-5-yl)-4,5-dimethoxy-methyl-β-carbolin (0,25 g). Schmelzpunkt 175 - 177 °C.

Analog werden hergestellt:

5,6,7,8-Tetrahydro-3-(3-ethyl-1,2,4-oxadiazol-5-yl)-4-methyl-5-(4-morpho-linyl)-methyl-β-carbolin. Schmelzpunkt 223 - 225 °C.

Beispiel 14

5,6,7,8-Tetrahydro-4-methoxymethyl-5-(4-morpholinyl)-methyl-β-carbolin-3-carbonsäure-ethylester

5,6,7,8-Tetrahydro-5-hydroxymethyl-4-methoxymethyl-β-carbolin-3-carbonsäure-ethylester (0,78 g) wird in waserfreiem Tetrahydrofuran (15 ml) gelöst (Lösung A).

Unter Argonschutz wird zu einer Lösung von Triphenylphosphin (3,2 g) in wasserfreiem Tetrahydrofuran (65 ml) bei 0 °C langsam Azodicarbonsäure-diethylester (2,1 g) gegeben. Nach 20 Minuten Rühren wird Lithiumbromid (2,11 g) zugegeben (Lösung B).

Bei 0 °C wird Lösung A zu Lösung B getropft. Das Gemisch wird erst eine Stunde bei 0 °C, dann 1 1 /2 Stunden bei Raumtemperatur gerührt. Zu der wieder auf 0 °C abgekühlten Mischung wird Morpholin (23,1 g) getropft. Nach 15 Stunden bei Raumtemperatur wird eingedampft, der Eindampfrückstand wird an Kieselgel mit einer Mischung aus 95 Teilen Dichlormethan und 5 Teilen Ethanol chromatographiert.

Der 5,6,7,8-Tetrahydro-4-methoxymethyl-5-(4-morpholinyl)-methyl-β-carbolin-3-carbonsäure-ethylester kristallisiert beim Anreiben mit Ether. Schmelzpunkt 188 - 191 0°.

Die Ausbeute beträgt 0,81 g.

Analog werden hergestellt:

5,6,7,8-Tetrahydro-4-methyl-5-(4-morpholinyl) methyl-β-carbolin-3-car-bonsäure-ethylester

Beispiel 15

5,6,7,8-Tetrahydro-5-ethoxymethyl-4-methoxymethyl-β-carbolin-3-carbonsäure

5,6,7,8-Tetrahydro-5-ethoxymethyl-4-methoxymethyl-β-carbolin-3-carbonsäure-ethylester (0,51 g) wird in Ethanol (25 ml) mit 1-normaler Natronlauge (4,3 ml) 3 Stunden am Rückfluß gekocht. Dann wird die Lösung mit 5,3 ml 1-normaler Salzsäure versetzt und eingedampft. Der Rückstand wird mit Ethanol ausgekocht, aus dem Ethanolextrakt erhält man nach Behandlung mit Kohle durch Eindampfen 0,49 g 5,6,7,8-Tetrahydro-5-ethoxymethyl-4-methoxy-methyl-β-carbolin-3-carbonsäure. Schmelzpunkt 250 °C (zers.).

Analog wird hergestellt:
5,6,7,8-Tetrahydro-4,5-dimethoxymethyl-β-carbolin-3-carbonsäure.

Außer den in den Beispielen genannten Verbindungen sind noch die folgenden Verbindungen besonders bevorzugt:
5,6,7,8-Tetrahydro-5-phenoxymethyl-β-carbolin-3-carbonsäure-ethylester
5,6,7,8-Tetrahydro-5-methoxymethyl-4-methyl-β-carbolin-3-carbonsäureisopropylamid.

**Patentansprüche**

**1.** 5,6,7,8-Tetrahydro-β-carbolinderivate der allgemeinen Formel I

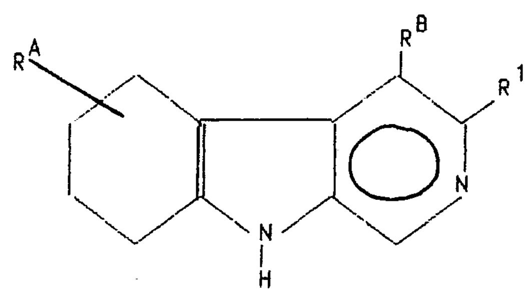

(I)

worin

$R^1$ einen Oxadiazolylrest der Formel

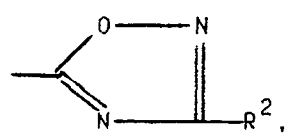

COOR$^3$ oder CONR$^4$R$^5$ bedeutet und
$R^2$ H, $C_{1-4}$-Alkyl oder $C_{3-6}$-Cycloalkyl,
$R^3$ H oder $C_{1-4}$-Alkyl.
$R^4$ und $R^5$ H, $C_{1-4}$-Alkyl oder Hydroxy-$C_{1-4}$-Alkyl darstellen, wobei $R^4$ und $R^5$ nicht gleichzeitig Wasserstoff sein können, oder $R^4$ und $R^5$ gemeinsam mit dem benachbarten Stickstoffatom einen Piperidin-, Morpholin-, Pyrrolidin-, Thiomorpholin oder Piperazin-Ring bilden und

$R^A$ Wasserstoff, =O, $C_{3-6}$-Cycloalkyl, -COOR$^3$ oder $C_{1-4}$-Alkyl, das gegeben enfalls durch OH, Halogen, $C_{1-4}$-Alkoxy, Phenyl, Phenoxy, -NR$^4$R$^5$, substituiert sein kann, wobei $R^3$, $R^4$ und $R^5$ die obige Bedeutung haben, und

$R^8$ Wasserstoff, $C_{1-4}$-Alkyl oder $C_{1-4}$-Alkoxyalkyl ist.

**2.** 5,6,7,8-Tetrahydro-5-ethoxymethyl-4-methyl-β-carbolin-3-carbonsäureethylester
5,6,7,8-Tetrahydro-5-methoxymethyl-4-methyl-β-carbolin-3-carbonsäureethylester
5,6,7,8-Tetrahydro-5-propoxymethyl-4-methyl-β-carbolin-3-carbonsäureethylester

5,6,7,8-Tetrahydro-4,5-dimethoxymethyl-β-carbolin-3-carbonsäureethylester
5,6,7,8-Tetrahydro-5-hydroxymethyl-4-methoxymethyl-β-carbolin-3-carbonsäureethylester
5,6,7,8-Tetrahydro-5-brommethyl-4-methoxymethyl-β-carbolin-3-carbonsäureethylester
5,6,7,8-Tetrahydro-5-ethoxymethyl-β-carbolin-3-carbonsäureethylester
5,6,7,8-Tetrahydro-5-ethoxymethyl-4-methoxymethyl-β-carbolin-3-carbonsäureethylester
5,6,7,8-Tetrahydro-5-hydroxymethyl-4-methyl-β-carbolin-3-carbonsäureethylester
5,6,7, 8-Tetrahydro-5-ethoxymethyl-4-ethyl-β-carbolin-3-carbonsäureethylester
5,6,7,8-Tetrahydro-5-phenoxymethyl-β-carbolin-3-carbonsäureethylester

**3.** 5,6,7,8-Tetrahydro-5-ethoxymethyl-4-methyl-β-carbolin-3-carbonsäuretert.-butylester
5,6,7,8-Tetrahydro-5-oxo-4-methoxymethyl-β-carbolin-3-carbonsäureisopropylester
5,6,7,8-Tetrahydro-5-ethyl-β-carbolin-3-carbonsäureisopropylester
5,6,7,8-Tetrahydro-5-(1-hydroxyethyl)-β-carbolin-3-carbonsäureisopropylester
5,6,7,8-Tetrahydro-5-methoxymethyl-4-methyl-β-carbolin-3-carbonsäureisopropylamid

**4.** 5,6,7,8-Tetrahydro-4-methoxymethyl-3-(3-ethyl-1,2,4-oxadiazol-5-yl)-β-carbolin
5,6,7,8-Tetrahydro-5-ethoxymethyl-3-(3-ethyl-1,2,4-oxadiazol-5-yl)-4-methyl-β-carbolin
5,6,7,8-Tetrahydro-4,5-dimethyl-3-(3-ethyl-1,2,4,-oxadiazol-5-yl)-β-carbolin
5,6,7,8-Tetrahydro-5-ethoxymethyl-3-(3-ethyl-1,2,4-oxadiazol-5-yl)-4-methoxymethyl-β-carbolin
5,6,7,8-Tetrahydro-4,5-dimethoxymethyl-3-(3-ethyl-1,2,4-oxadiazol-5-yl)-β-carbolin
5,6,7,8-Tetrahydro-4-methyl-5-(4-morpholinyl)-methyl-3-(3-ethyl-1,2,4-oxadiazol-5-yl)-β-carbolin
5,6,7, 8-Tetrahydro-4-methoxymethyl-5-(4-morpholinyl)-methyl-3-(3-ethyl-1,2,4-oxadiazol-5-yl)-β-carbolin
5,6,7,8-Tetrahydro-4-methyl-5-methoxymethyl--3-(3-ethyl, 1,2,4-oxadiazol-5-yl)-β -carbolin

**5.** 5,6,7,8-Tetrahydro-4-methoxymethyl-β-carbolin-3-carbonsäureethylester
5,6,7,8-Tetrahydro-β-carbolin-3-carbonsäureethylester
5,6,7,8-Tetrayhdro-4-ethyl-β-carbolin-3-carbonsäureethylester
5,6,7,8-Tetrahydro-4-methyl-β-carbolin-3-carbonsäureethylester

**6.** 5,6,7,8-Tetrahydro-5-ethoxymethyl-4-methoxymethyl-β-carbolin-3-carbonsäure
5,6,7,8-Tetrahydro-5-ethoxymethyl-4-methyl-β-carbolin-3-carbonsäure
5,6,7,8-Tetrahydro-5-(4-morpholinyl)-methyl-4-methoxymethyl-β-carbolin-3-carbonsäure
5,6,7, 8-Tetrahydro-5-(4-morpholinyl)-methyl-4-methyl-β-carbolin-3-carbonsäure
5,6,7,8-Tetrahydro-4-methoxymethyl-β-carbolin-3-carbonsäure
5,6,7,8-Tetrahydro-4,5-dimethyl-β-carbolin-3-carbonsäure
5,6,7,8-Tetrahydro-5-methoxymethyl-4-methyl-β-carbolin-3-carbonsäure
5,6,7,8-Tetrahydro-4,5-dimethoxymethyl-β-carbolin-3-carbonsäure

**7.** 5,6,7,8-Tetrahydro-5-(4-morpholinyl)-methyl-4-methoxymethyl-β-carbolin-3-carbonsäureethylester
5,6,7,8-Tetrahydro-5-diethylaminomethyl-4-methoxymethyl-β-carbolin-3-carbonsäureethylester
5,6,7,8-Tetrahydro-5-diethanolaminomethyl-4-methoxymethyl-β-carbolin-3-carbonsäureethylester
5,6,7,8-Tetrahydro-5-isopropylaminomethyl-4-methoxymethyl-β-carbolin-3-carbonsäureethylester
5,6.7,8-Tetrahydro-5-(4-morpholinyl)-methyl-4-methyl-β-carbolin-3-carbonsäureethylester

**8.** 5,6,7,8-Tetrahydro-β-carbolin-3,5-dicarbonsäurediethylester
5,6,7,8-Tetrayhdro-6-benzyl-4-methyl-β-carbolin-3-carbonsäureethylester
5,6,7,8-Tetrahydro-6-cyclohexyl-4-methyl-β-carbolin-3-carbonsäureethylester
5,6,7,8-Tetrahydro-5-oxo-4-methoxymethyl-β-carbolin-3-carbonsäureethylester
5,6,7,8-Tetrahydro-5-oxo-4-methyl-β-carbolin-3-carbonsäure-ethylester
5,6,7,8-Tetrahydro-4,5-dimethyl-β-carbolin-3-carbonsäureethylester

**9.** Verfahren zur Herstellung der Verbindungen der allgemeinen Formel I, dadurch gekennzeichnet, daß man in an sich bekannter Weise
eine Verbindung der allgemeinen Formel II

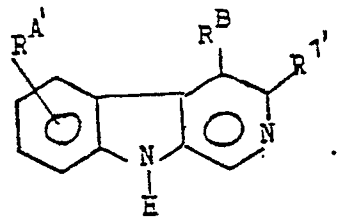

(II),

worin

$R^B$ die oben genannte Bedueutung hat,

$R^{1'}$ $-COOR^3$ darstellt mit $R^3$ in der oben genannten Bedeutung und

$R^{A'}$ Wasserstoff, eine Hydroxygruppe, $C_3$-$C_6$ Cycloalkyl, $-COOR^3$, worin $R^3$ die oben genannte Bedeutung hat, oder $C_{1-4}$ Alkyl, das mit Phenyl, $C_{1-4}$ Alkoxy oder Hydroxy substituiert sein kann, bedeutet, in Gegenwart eines Katalysators hydriert und gegebenenfalls anschließend Verbindungen der allgemeinen Formel I mit $R^A$ in der Bedeutung einer Hydroxyalkylgruppe mit Phenol veräthert oder die Hydroxygruppe durch sekundäre Amine oder durch Halogen substituiert und anschließend gegebenenfalls die so erhaltene Halogenalkylverbindung mit einem Amin der Formel $HNR^4R^5$ mit $R^4$ und $R^5$ in der oben genannten Bedeutung aminiert oder eine Estergruppe umestert, amidiert oder hydrolisiert und gegebenenfalls anschließend die so erhaltene freie Säure amidiert oder in den tert.-Butylester überführt oder mit einem Amidoxim der Formel $R^2$-C(=NOH)$NH_2$ mit $R^2$ in der oben genannten Bedeutung umsetzt.

**10.** Verwendung der Verbindungen gemäß Ansprüche 1 bis 8 als Arzneimittel.

## Claims

**1.** 5,6,7,8-Tetrahydro-β-carboline derivatives of the general formula I

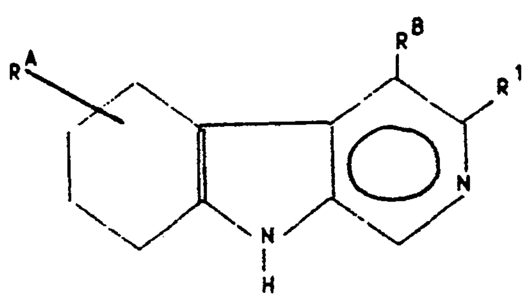

(I)

in which

$R^1$ represents an oxadiazolyl radical of the formula

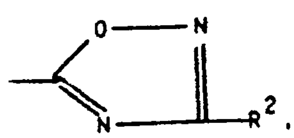

$COOR^3$ or $CONR^4R^5$ and

$R^2$ represents H, $C_{1-4}$alkyl or $C_{3-6}$cycloalkyl,

$R^3$ represents H or $C_{1-4}$alkyl,

$R^4$ and $R^5$ represent H, $C_{1-4}$alkyl or hydroxy-$C_{1-4}$alkyl but $R^4$ and $R^5$ cannot simultaneously be hydrogen, or $R^4$ and $R^5$ together with the adjacent nitrogen atom form a piperidine, morpholine, pyrrolidine, thiomorpholine or piperazine ring, and

$R^A$ represents hydrogen, =O, $C_{3-6}$cycloalkyl, $-COOR^3$ or $C_{1-4}$alkyl, which may optionally be substituted by OH, halogen, $C_{1-4}$alkoxy, phenyl, phenoxy, or by $-NR^4R^5$, $R^3$, $R^4$ and $R^5$

having the meanings given above, and

$R^B$ represents hydrogen, $C_{1-4}$alkyl or $C_{1-4}$alkoxyalkyl.

2. 5,6,7,8-Tetrahydro-5-ethoxymethyl-4-methyl-β-carboline-3-carboxylic acid ethyl ester
5,6,7,8-tetrahydro-5-methoxymethyl-4-methyl-β-carboline-3-carboxylic acid ethyl ester
5,6,7,8-tetrahydro-5-propoxymethyl-4-methyl-β-carboline-3-carboxylic acid ethyl ester
5,6,7,8-tetrahydro-4,5-dimethoxymethyl-β-carboline-3-carboxylic acid ethyl ester
5,6,7,8-tetrahydro-5-hydroxymethyl-4-methoxymethyl-β-carboline-3-carboxylic acid ethyl ester
5,6,7,8-tetrahydro-5-bromomethyl-4-methoxymethyl-β-carboline-3-carboxylic acid ethyl ester
5,6,7,8-tetrahydro-5-ethoxymethyl-β-carboline-3-carboxylic acid ethyl ester
5,6,7,8-tetrahydro-5-ethoxymethyl-4-methoxymethyl-β-carboline-3-carboxylic acid ethyl ester
5,6,7,8-tetrahydro-5-hydroxymethyl-4-methyl-β-carboline-3-carboxylic acid ethyl ester
5,6,7,8-tetrahydro-5-ethoxymethyl-4-ethyl-β-carboline-3-carboxylic acid ethyl ester
5,6,7,8-tetrahydro-5-phenoxymethyl-β-carboline-3-carboxylicacid ethyl ester.

3. 5,6,7,8-Tetrahydro-5-ethoxymethyl-4-methyl-β-carboline-3-carboxylic acid tert.-butyl ester
5,6,7,8-tetrahydro-5-oxo-4-methoxymethyl-β-carboline-3-carboxylic acid isopropyl ester
5,6,7,8-tetrahydro-5-ethyl-β-carboline-3-carboxylic acid isopropyl ester
5,6,7,8-tetrahydro-5-(1-hydroxyethyl)-β-carboline-3-carboxylic acid isopropyl ester
5,6,7,8-tetrahydro-5-methoxymethyl-4-methyl-β-carboline-3-carboxylic acid isopropyl amide.

4. 5,6,7,8-Tetrahydro-4-methoxymethyl-3-(3-ethyl-1,2,4-oxadiazol-5-yl)-β-carboline
5,6,7,8-tetrahydro-5-ethoxymethyl-3-(3-ethyl-1,2,4-oxadiazol-5-yl)-4-methyl-β-carboline
5,6,7,8-tetrahydro-4,5-dimethyl-3-(3-ethyl-1,2,4-oxadiazol-5-yl)-β-carboline
5,6,7,8-tetrahydro-5-ethoxymethyl-3-(3-ethyl-1,2,4-oxadiazol-5-yl)-4-methoxymethyl-β-carboline
5,6,7,8-tetrahydro-4,5-dimethoxymethyl-3-(3-ethyl-1,2,4-oxadiazol-5-yl)-β-carboline
5,6,7,8-tetrahydro-4-methyl-5-(4-morpholinyl)-methyl-3-(3-ethyl-1,2,4-oxadiazol-5-yl)-β-carboline
5,6,7,8-tetrahydro-4-methoxymethyl-5-(4-morpholinyl)-methyl-3-(3-ethyl-1,2,4-oxadiazol-5-yl)-β-carboline
5,6,7,8-tetrahydro-4-methyl-5-methoxymethyl-3-(3-ethyl-1,2,4-oxadiazol-5-yl)-β-carboline.

5. 5,6,7,8-Tetrahydro-4-methoxymethyl-β-carboline-3-carboxylicacid ethyl ester
5,6,7,8-tetrahydro-β-carboline-3-carboxylic acid ethyl ester
5,6,7,8-tetrahydro-4-ethyl-β-carboline-3-carboxylic acid ethyl ester
5,6,7,8-tetrahydro-4-methyl-β-carboline-3-carboxylic acid ethyl ester.

6. 5,6,7,8-Tetrahydro-5-ethoxymethyl-4-methoxymethyl-β-carboline-3-carboxylic acid
5,6,7,8-tetrahydro-5-ethoxymethyl-4-methyl-β-carboline-3-carboxylic acid
5,6,7,8-tetrahydro-5-(4-morpholinyl)-methyl-4-methoxymethyl-β-carboline-3-carboxylic acid
5,6,7,8-tetrahydro-5-(4-morpholinyl)-methyl-4-methyl-β-carboline-3-carboxylic acid
5,6,7,8-tetrahydro-4-methoxymethyl-β-carboline-3-carboxylicacid
5,6,7,8-tetrahydro-4,5-dimethyl-β-carboline-3-carboxylic acid
5,6,7,8-tetrahydro-5-methoxymethyl-4-methyl-β-carboline-3-carboxylic acid
5,6,7,8-tetrahydro-4,5-dimethoxymethyl-β-carboline-3-carboxylic acid.

7. 5,6,7,8-Tetrahydro-5-(4-morpholinyl)-methyl-4-methoxymethyl-β-carboline-3-carboxylic acid ethyl ester
5,6,7,8-tetrahydro-5-diethylaminomethyl-4-methoxymethyl-β-carboline-3-carboxylic acid ethyl ester
5,6,7,8-tetrahydro-5-diethanolaminomethyl-4-methoxymethyl-β-carboline-3-carboxylic acid ethyl ester
5,6,7,8-tetrahydro-5-isopropylaminomethyl-4-methoxymethyl-β-carboline-3-carboxylic acid ethyl ester
5,6,7,8-tetrahydro-5-(4-morpholinyl)-methyl-4-methyl-β-carboline-3-carboxylic acid ethyl ester.

8. 5,6,7,8-Tetrahydro-β-carboline-3,5-dicarboxylic acid diethyl ester
5,6,7,8-tetrahydro-6-benzyl-4-methyl-β-carboline-3-carboxylic acid ethyl ester
5,6,7,8-tetrahydro-6-cyclohexyl-4-methyl-β-carboline-3-carboxylic acid ethyl ester
5,6,7,8-tetrahydro-5-oxo-4-methoxymethyl-β-carboline-3-carboxylic acid ethyl ester
5,6,7,8-tetrahydro-5-oxo-4-methyl-β-carboline-3-carboxylic acid ethyl ester
5,6,7,8-tetrahydro-4,5-dimethyl-β-carboline-3-carboxylic acid ethyl ester.

9. A process for the manufacture of compounds of the general formula I, characterised in that in a manner

known per se,
a compound of the general formula II

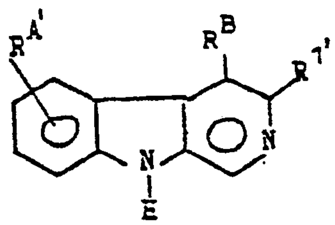

(II)

in which
$R^B$ has the meaning given above,
$R^{1'}$ represents $-COOR^3$ in which $R^3$ has the meaning given above, and
$R^{A'}$ represents hydrogen, a hydroxy group,
$C_3$-$C_6$cycloalkyl, $-COOR^3$ in which $R^3$ has the meaning given above, or $C_{1-4}$alkyl, which may be substituted by phenyl, $C_{1-4}$alkoxy or by hydroxy, is hydrogenated in the presence of a catalyst, and optionally then compounds of the general formula I in which $R^A$ represents a hydroxyalkyl group are etherified with phenol or the hydroxy group is substituted by secondary amines or by halogen, and then optionally the haloalkyl compound so obtained is aminated with an amine of the formula $HNR^4R^5$ in which $R^4$ and $R^5$ have the meanings given above, or an ester group is transesterified, amidated or hydrolysed, and optionally then the free acid so obtained is amidated or converted into the tert.-butyl ester or reacted with an amide oxime of the formula $R^2$-C(=NOH)$NH_2$ in which $R^2$ has the meaning given above.

**10.** The use of compounds according to claims 1 to 8 as medicaments.

**Revendications**

**1.** Tétrahydro-5,6,7,8 β-carbolines qui répondent à la formule générale I :

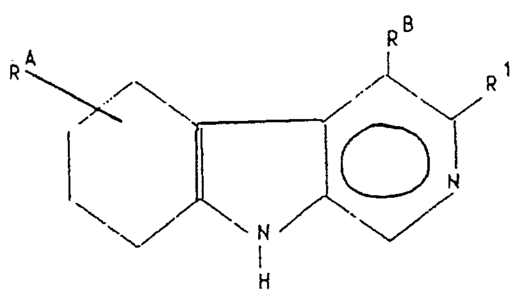

(I)

dans laquelle
$R^1$ représente un radical oxadiazolyle de formule

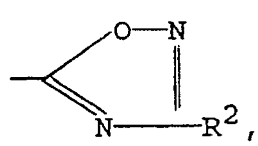

un radical $-COOR^3$ ou un radical $-CONR^4R^5$, radicaux dans lesquels :
$R^2$ représente H, un alkyle en $C_1$-$C_4$ ou un cycloalkyle en $C_3$-$C_6$,
$R^3$ représente H ou un alkyle en $C_1$-$C_4$,
$R^4$ et $R^5$ représentent chacun H, un alkyle en $C_1$-$C_4$ ou un hydroxy-alkyle en $C_1$-$C_4$, $R^4$ et $R^5$ ne pouvant pas représenter en même temps chacun l'hydrogène, ou $R^4$ et $R^5$ forment ensemble, et avec l'atome d'azote voisin, un cycle de pipéridine, de morpholine, de

pyrrolidine, de thiomorpholine ou de pipérazine,

$R^A$ représente l'hydrogène, =O, un cycloalkyle en $C_3$-$C_6$, un radical $-COOR^3$ ou un alkyle en $C_1$-$C_4$ éventuellement porteur d'un radical OH, d'un halogène, d'un alcoxy en $C_1$-$C_4$, d'un phényle, d'un phénoxy ou d'un radical $-NR^4R^5$, les symboles $R^3$, $R^4$ et $R^5$ ayant les significations indiquées ci-dessus, et

$R^B$ représente l'hydrogène, un alkyle en $C_1$-$C_4$ ou un alcoxy-alkyle en $C_1$-$C_4$.

**2.** Tétrahydro-5,6,7,8 éthoxyméthyl-5 méthyl-4 $\beta$-carboline-carboxylate-3 d'éthyle

Tétrahydro-5,6,7,8 méthoxyméthyl-5 méthyl-4 $\beta$-carboline-carboxylate-3 d'éthyle

Tétrahydro-5,6,7,8 propoxyméthyl-5- méthyl-4 $\beta$-carboline-carboxylate-3 d'éthyle

Tétrahydro-5,6,7,8 bis-(méthoxyméthyl)-4,5 $\beta$-carboline-carboxylate-3 d'éthyle

Tétrahydro-5,6,7,8 hydroxyméthyl-5 méthoxyméthyl-4 $\beta$-carboline-carboxylate-3 d'éthyle

Tétrahydro-5,6,7,8 bromométhyl-5 méthoxyméthyl-4 $\beta$-carboline-carboxylate-3 d'éthyle

Tétrahydro-5,6,7,8 éthoxyméthyl-5 $\beta$-carboline-carboxylate-3 d'éthyle

Tétrahydro-5,6,7,8 éthoxyméthyl-5 méthoxyméthyl-4 $\beta$-carboline-carboxylate-3 d'éthyle

Tétrahydro-5,6,7,8 hydroxyméthyl-5 méthyl-4 $\beta$-carboline-carboxylate-3 d'éthyle

Tétrahydro-5,6,7,8 éthoxyméthyl-5 éthyl-4 $\beta$-carboline-carboxylate-3 d'éthyle

Tétrahydro-5,6,7,8 phénoxyméthyl-5 $\beta$-carboline-carboxylate-3d'éthyle.

**3.** Tétrahydro-5,6,7,8 éthoxyméthyl-5 méthyl-4 $\beta$-carboline-carboxylate-3 de tert-butyle

Tétrahydro-5,6,7,8 oxo-5 méthoxyméthyl-4 $\beta$-carboline-carboxylate-3 d'isopropyle

Tétrahydro-5,6,7,8 éthyl-5 $\beta$-carboline-carboxylate-3 d'isopropyle

Tétrahydro-5,6,7,8 (hydroxy-1 éthyl)-5- $\beta$-carboline-carboxylate-3 d'isopropyle

Isopropylamide de l'acide tétrahydro-5,6,7,8 méthoxyméthyl-5 méthyl-4 $\beta$-carboline-carboxylique-3.

**4.** Tétrahydro-5,6,7,8 méthoxyméthyl-4 (éthyl-3 oxadiazole-1,2,4 yl-5)-3 $\beta$-carboline

Tétrahydro-5,6,7,8 éthoxyméthyl-5 (éthyl-3 oxadiazole-1,2,4 yl-5)-3 méthyl-4 $\beta$-carboline

Tétrahydro-5,6,7,8 diméthyl-4,5 (éthyl-3 oxadiazole-1,2,4 yl-5)-3 $\beta$-carboline

Tétrahydro-5,6,7,8 éthoxyméthyl-5 (éthyl-3 oxadiazole-1,2,4 yl-5)-3 méthoxyméthyl-4 $\beta$-carboline

Tétrahydro-5,6,7,8 bis-(méthoxyméthyl)-4,5 (éthyl-3 oxadiazole-1,2,4 yl-5)-3 $\beta$-carboline

Tétrahydro-5,6,7,8 méthyl-4 [(morpholinyl-4)-méthyl]-5 (éthyl-3 oxadiazole-1,2,4 yl-5)-3 $\beta$-carboline

Tétrahydro-5,6,7,8 méthoxyméthyl-4 [(morpholinyl-4)-méthyl]-5 (éthyl-3 oxadiazole-1,2,4 yl-5)-3 $\beta$-carboline

Tétrahydro-5,6,7,8 méthyl-4 méthoxyméthyl-5 (éthyl-3 oxadiazole-1,2,4 yl-5)-3 $\beta$-carboline.

5. Tétrahydro-5,6,7,8 méthoxyméthyl-4 β-carboline-carboxylate-3d'éthyle

Tétrahydro-5,6,7,8 β-carboline-carboxylate-3 d'éthyle

Tétrahydro-5,6,7,8 éthyl-4 β-carboline-carboxylate-3 d'éthyle

Tétrahydro-5,6,7,8 méthyl-4 β-carboline-carboxylate-3 d'éthyle

6. Acide tétrahydro-5,6,7,8 éthoxyméthyl-5 méthoxyméthyl-4 β-carboline-carboxylique-3

Acide tétrahydro-5,6,7,8 éthoxyméthyl-5 méthyl-4 β-carboline-carboxylique-3

Acide tétrahydro-5,6,7,8 [(morpholinyl-4)-méthyl]-5 méthoxyméthyl-4 β-carboline-carboxylique-3

Acide tétrahydro-5,6,7,8 [(morpholinyl-4)-méthyl]-5 méthyl-4 β-carboline-carboxylique-3

Acide tétrahydro-5,6,7,8 méthoxyméthyl-4 β-carboline-carboxylique-3

Acide tétrahydro-5,6,7,8 diméthyl-4,5 β-carboline-carboxylique-3

Acide tétrahydro-5,6,7,8 méthoxyméthyl-5 méthyl-4 β-carboline-carboxylique-3

Acide tétrahydro-5,6,7,8 bis-(méthoxyméthyl)-4,5 β-carboline-carboxylique-3.

7. Tétrahydro-5,6,7,8 [(morpholinyl-4)-méthyl]-5 méthoxyméthyl-4 β-carboline-carboxylate-3 d'éthyle

Tétrahydro-5,6,7,8 diéthylaminométhyl-5 méthoxyméthyl-4 β-carboline-carboxylate-3 d'éthyle

Tétrahydro-5,6,7,8 diéthylaminométhyl-5 méthoxyméthyl-4 β-carboline-carboxylate-3 d'éthyle

Tétrahydro-5,6,7,8 isopropylaminométhyl-5 méthoxyméthyl-4 β-carboline-carboxylate-3 d'éthyle

Tétrahydro-5,6,7,8 [(morpholinyl-4)-méthyl]-5 méthyl-4 β-carboline-carboxylate-3 d'éthyle.

8. Tétrahydro-5,6,7,8 β-carboline-dicarboxylate-3,5 de diéthyle

Tétrahydro-5,6,7,8 benzyl-6 méthyl-4 β-carboline-carboxylate-3d'éthyle

Tétrahydro-5,6,7,8 cyclohexyl-6 méthyl-4 β-carboline-carboxylate-3 d'éthyle.

Tétrahydro-5,6,7,8 oxo-5 méthoxyméthyl-4 β-carboline-carboxylate-3 d'éthyle

Tétrahydro-5,6,7,8 oxo-5 méthyl-4 β-carboline-carboxylate-3d'éthyle

Tétrahydro-5,6,7,8 diméthyl-4,5 β-carboline-carboxylate-3 d'éthyle.

9. Procédé pour préparer les composés de formule générale I, procédé caractérisé en ce que, en opérant de manière connue, on hydrogène en présence d'un catalyseur un composé répondant à la formule générale II :

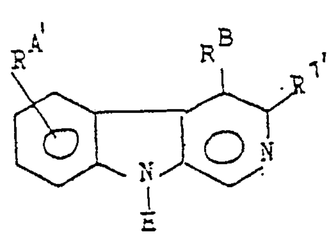

(II),

dans laquelle

$R^B$ a la signification qui lui a été donnée ci-dessus,

$R^{1'}$ représente un radical $-COOR^3$ dans lequel $R^3$ a la signification qui lui a été donnée ci-dessus, et

$R^{A'}$ représente l'hydrogène, un hydroxy, un cycloalkyle en $C_3$-$C_6$, un radical $-COOR^3$ dans lequel $R^3$ a la signification qui lui a été donnée ci-dessus, ou un radical alkyle en $C_1$-$C_4$ éventuellement porteur d'un phényl, d'un alcoxy en $C_1$-$C_4$ ou d'un hydroxy,

puis, le cas échéant, on éthérifie par le phénol des composés de formule générale I dans lesquels $R^A$ représente un radical hydroxy-alkyle ou on remplace le radical hydroxy par celui d'une amine secondaire ou par un halogène, après quoi, le cas échéant, on amine le composé halogéno-alkylique ainsi obtenu avec une amine de formule $HNR^4R^5$ dans laquelle $R^4$ et $R^5$ ont les significations indiquées ci-dessus, ou on transestérifie, amide ou hydrolyse un radical d'ester, puis, éventuellement, on amide l'acide libre ainsi obtenu ou on le transformer en l'ester tert-butylique ou on le fait réagir avec une amide-oxime de formule $R^2$-$C(=NOH)NH_2$ dans laquelle $R^2$ a la signification qui lui a été donnée plus haut.

**10.** Application des composés selon l'une quelconque des revendications 1 à 8 comme médicaments.